# EUROPÄISCHE PATENTSCHRIFT

(11) **EP 0 448 912 B1**
(45) Veröffentlichungstag und Bekanntmachung des Hinweises auf die Patenterteilung: **28.08.1996**
(21) Anmeldenummer: 90810870.7
(22) Anmeldetag: 12.11.1990
(51) Int. Cl.: C12M 1/26

(54) **Anlage zur automatischen, periodischen Probe-Entnahme aus einem Bioreaktor-Fermenter**
Plant for automatic and periodical sampling of the content of a bioreactor-fermentor
Installation pour l'échantillonnage automatique et périodique du contenu d'un bioréacteur-fermenteur.

(30) Priorität: 30.03.1990 CH 1070/90
(43) Veröffentlichungstag der Anmeldung: 02.10.1991
(73) Patentinhaber: Paluschinski, Hans, CH-8311 Brütten (CH)
(72) Erfinder: Paluschinski, Hans, CH-8311 Brütten (CH)
(74) Vertreter: Feldmann, Clarence Paul

(56) Entgegenhaltungen:
- CH-A- 475 350
- FR-A- 1 405 957
- GB-A- 1 215 086
- BIOTECHNOLOGY & BIOENGINEERING, Band 32, Nr. 7, 20 September 1988, New York, NY
- (US); G.K.E. SEIFFERT et al., Seiten 923-926/

## Beschreibung

Biologische Prozesse unterliegen Bedingungen, die die Lebensfähigkeit der Mikroorganismen nicht einschränken dürfen.

Der Ablauf einer Fermentation lässt sich nicht durch extreme Prozessparameter beschleunigen.
Ein Fermentations-Zyklus benötigt deshalb eine relativ grosse Zeitspanne (mehrere Stunden oder Tage).

Den Prozessablauf bei der Erarbeitung neuer Verfahren im Labor oder bei der Durchführung in industriellen Massstab muss in seinem Ablauf beobachtet werden. Deshalb werden in gewissen Zeitabständen oder bedingt durch Prozessparameter Proben aus dem Fermenter-Inhalt entnommen und untersucht. In diese zeitraubende Prozedur der Proben-Entnahme war bisher das Laborpersonal stark einbezogen. Da der Prozess-Zeitraum die normale Arbeitszeit überschreitet, waren bislang Sonder- oder Nachtschichten notwendig. Andernfalls musste der Prozess entsprechend wiederholt werden, um unkontrollierte Lücken zu schliessen und so einen kontinuierlichen Ueberblick über den Prozessablauf zu erhalten.

Aus Biotechnology Bioengineering Vol. 32 p.923-926 (1988) ist zwar eine automatische Probeentnahme bekannt. Diese Anlage weist ein Dreiwegventil auf. Die Reagenzgläser sind stationär in einem Kühlbehälter angeordnet, wobei die Probenverteilung in die einzelnen Reagenzgläser über eine programmierbare Verteilvorrichtung erfolgt, die mit jedem Reagenzglas mit einer separaten Zuleitung verbunden ist.

Die Erfindung hat sich zur Aufgabe gestellt, eine Anlage zu schaffen, welche automatisch, periodisch eine Probe-Entnahme aus einem Bioreaktor-Fermenter und eine Speicherung der entnommenen Proben vornimmt. Diese Anlage zeichnet sich durch die im kennzeichnenden Teil des Patentanspruches 1 genannten Merkmale aus.

Die Erfindung geht dabei von der Erkenntnis aus, dass Kälte den biochemischen Prozess stoppt. Die entnommenen Proben werden deshalb in einem gekühlten Aufnahmebehälter untergebracht und bleiben somit im Original-Entnahmezustand erhalten. Die Probeuntersuchungen können dann zu einem späteren Zeitpunkt während der normalen Arbeitszeit erfolgen. Es ist dabei von Vorteil, dass die Untersuchung der Proben in einem Arbeitsgang durchgeführt werden kann.

Die Entnahme der Proben ist ein "Handling-Verfahren", wobei allerdings einige spezielle Massnahmen zu beachten sind. Beispielsweise ändert sich die Viskosität der aus dem Fermenter zu entnehmenden Flüssigkeit, was die Fülldauer der Reagenzgläser beeinflusst. Eine Programmierung des Verfahrens erlaubt auch, Proben in gleichen Zeitintervallen oder in Zeitintervallen abhängig von den im Fermenter herrschenden Bedingungen vorzunehmen. Die Entnahme-Zeitintervalle können in Abhängigkeit der Viskosität der Flüssigkeit, das heisst in Abhängigkeit der Fülldauer eines Reagenzglases, gesteuert werden.

An Hand der beigefügten Zeichnung wird die erfindungsgemässe Anlage erläutert.

Die Zeichnung zeigt:
- Figur 1: eine vereinfachte Darstellung einer Anlage zur automatischen, periodischen Probe-Entnahme aus einem Bioreaktor-Fermenter:
- Figur 2: einen gekühlten Probe-Aufnahmebehälter im Schnitt;
- Figur 3: denselben Probe-Aufnahmebehälter in Ansicht von oben,
- Figur 4: ein fernbetätigbares Probe-Entnahmeventil;
- Figur 5: ein Reagenzglas während der Kontrolle des Füllstandes;
- Figur 6: eine Hebevorrichtung in drei verschiedenen Betriebslagen;
- Figur 7: ein mit der Hebevorrichtung verbundener Greifer für sich.

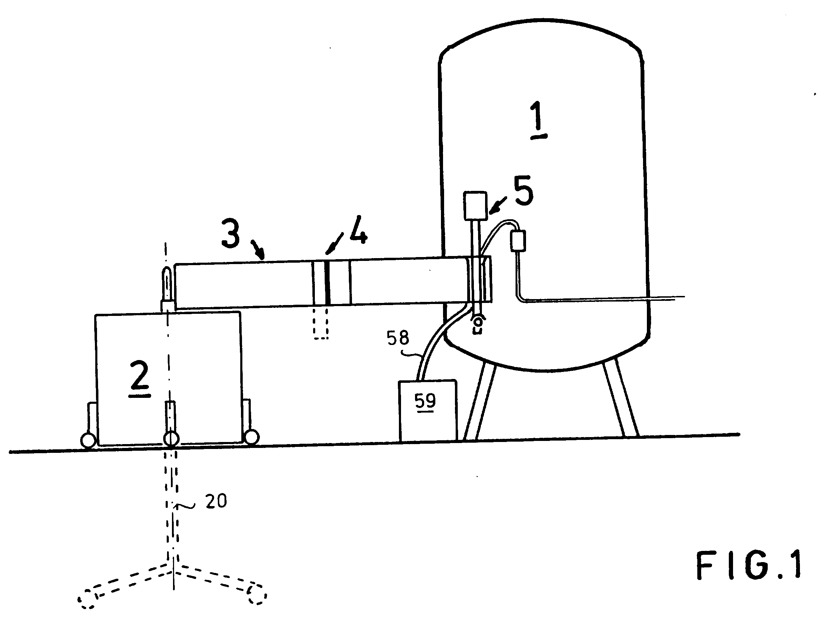

In Figur 1 sind sämtliche Teile der Anlage ersichtlich, die nachfolgend im Detail beschrieben werden.

Die Hauptbestandteile sind:
ein Fermenter 1, ein Probe-Aufbewahrungsbehälter 2 und eine den Fermenter 1 mit dem Behälter 2 verbindende Schiene 3, auf der ein Läufer 4 fahrbar geführt ist. Am Fermenter ist eine Probe-Entnahmevorrichtung 5 angebracht.

In Figur 1 ist eine Situation dargestellt, bei der der Aufnahmebehälter 2 auf derselben Standfläche wie der Fermenter 1 steht und trotzdem die Schiene 3 horizontal verläuft. Dies ist nicht immer möglich. Der Behälter 2 kann daher mit einer Vorrichtung 20 zur Höhenanpassung versehen sein, wie dies durch unterbrochene Linien in Figur 1 dargestellt ist.

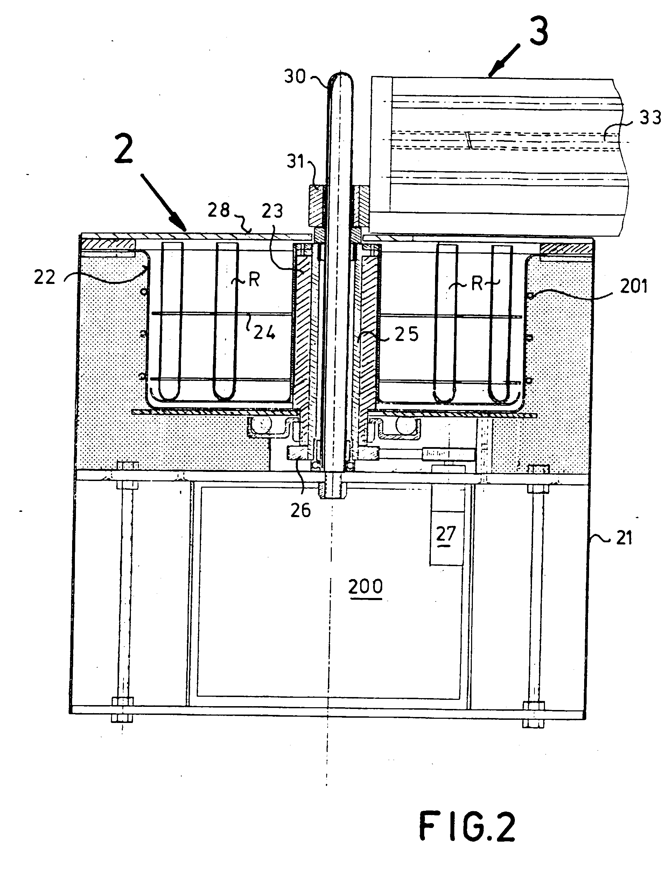

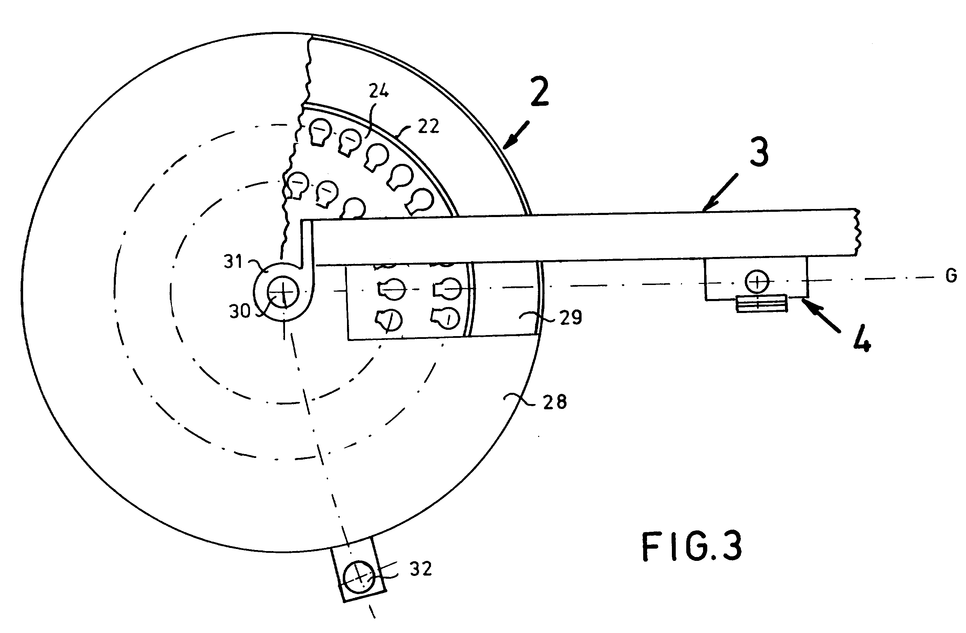

Die Figuren 2 und 3 zeigen den Probe-Aufbewahrungsbehälter 2 für sich. In diesen Figuren ist auch ein Teil der Schiene 3 und des Läufers 4 sichtbar. Der Aufbewahrungsbehälter umfasst ein Gehäuse 21, in dessen oberen Teil sich eine thermisch isolierte Wanne 22 mit einer zentralen Säule 23 befindet, die von einem Zentrierzapfen 30 durchsetzt ist. In der Wanne 22 ist ein Reagenzglas-Probehalter 24 gelagert, der um die Säule 23 drehbar gelagert ist. Der Probehalter ist mittels einer in der Säule gelagerten Hohlwelle 25 drehbar. Die Hohlwelle ist dazu unten mittels eines Zahnrades 26, eines Zahnriemens und eines Stellmotors 27 verbunden, der den Probehalter jeweils um einen Schritt weiter dreht, damit ein benachbartes im Probehalter gehaltenes Reagenzglas R genau auf die Bahnlinie G, auf der ein noch zu beschreibender Greifer läuft, zu liegen kommt.
In der unteren Gehäusehälfte ist ein Kühlaggregat 200 untergebracht, dessen Kühlrohre 201 aussen an der Aussenwand der Wanne 22 anliegen.
Von oben ist der Behälter mit einem Deckel 28 abgedeckt, der mit einer Oeffnung 29 versehen ist, durch welche ein Reagenzglas R entnommen beziehungsweise wieder in den Probehalter 24 eingesetzt wird.

Die Schiene 3, welche den Fermenter 1 mit dem Aufbewahrungsbehälter 2 verbindet, ist an der Seite des Behälters auf dem Zentrierzapfen 30 gehalten. Dazu dient eine an der Schiene 3 befestigte Oese 31. Am anderen Ende ist die Schiene an einem, mit dem Fermenter beziehungsweise der Entnahmevorrichtung verbundenen Kugelgelenk 36 gehalten wie später beschrieben wird. Diese Anordnung ermöglicht es, die Schiene vom Zentrierzapfen abzuheben, zu schwenken und auf einen seitlich am Behälter 2 angebrachten Zapfen 32 abzustellen.
In dieser "Parkstellung" der Schiene kann der Deckel 28 abgehoben und der Reagenzglasbehälter 24 mit den Reagenzgläsern aus der Wanne 22 herausgenommen werden.

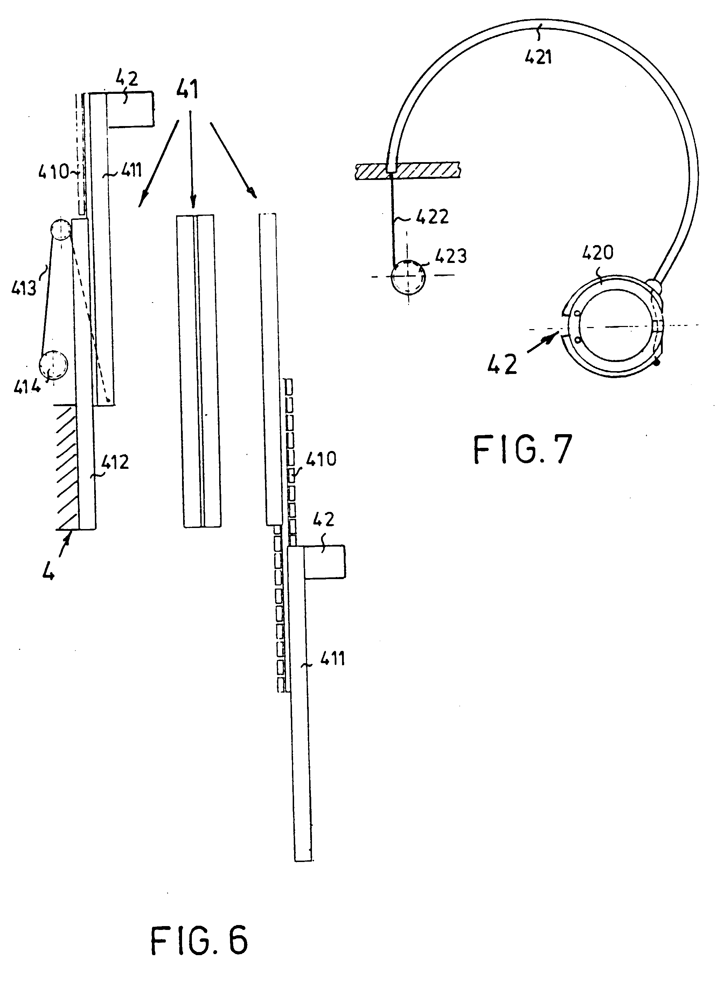

Der Läufer 4 ist gleitend auf der Schiene 3 geführt und wird mit Hilfe einer Spindel 33 mit Gewinde, die von einem nicht dargestellten Motor angetrieben ist, entlang der Schiene bewegt. Am Läufer 4 ist eine Hebevorrichtung 41 befestigt (Figur 6), die an ihrem oberen Ende einen Greifer 42 (Figur 7) für ein Reagenzglas R trägt.

Die Hebevorrichtung 41 ist als reibungslos verschiebbare Kulisse mit einem mittleren Rollenführungsteil 410 ausgebildet. Die Rollen laufen in Nuten, die in den Kulissenhälften 411, 412 angebracht sind. Die eine Kulissenhälfte 412 ist mit dem Läufer 4 verbunden, während die Kulissenhälfte 411 unten den Greifer 42 trägt. Die Auf- und Abwärtsbewegung der Kulissenhälfte 411 mit dem Greifer erfolgt über einen Seilzug 414, der auf einer kleinen motorisch betriebenen Seiltrommel 413 das Seil auf- oder abwickelt. Die Abwärtsbewegung erfolgt lediglich durch die Schwerkraft.

Tritt bei dieser Anordnung ein Fehler auf, beispielsweise wenn ein Reagenzglas in den Behälter an einer Stelle, die bereits besetzt ist, abgesenkt wird, hängt sofort das Seil 413 schlaff. Eine nicht dargestellte, am Seil mit leichtem Druck anliegende Blattfeder ändert dann ihre Lage, die mittels einer Lichtschranke überwacht wird. Diese gibt dann ein Signal ab, welches die Anlage stoppt.

In Figur 6 ist die Hebevorrichtung in drei Stellungen dargestellt, links in voll angehobenem Zustand, in der Mitte in einer Mittenlage und rechts in abgesenkter Lage. Der Antriebsmotor, welcher die Seiltrommel 413 treibt, erlaubt eine Fernsteuerung der Auf- oder Abwärtsbewegung.

Der Greifer 42 umfasst zwei gelenkig miteinander verbundene Hälften 420, die von einer nicht dargestellten Feder in geöffneter Lage wie in Figur 7 gehalten werden. Mittels eines die Oeffnung überbrückenden Bowden-Seilzuges können sie näher zueinander gebracht werden, so dass sie ein Reagenzglas fassen.

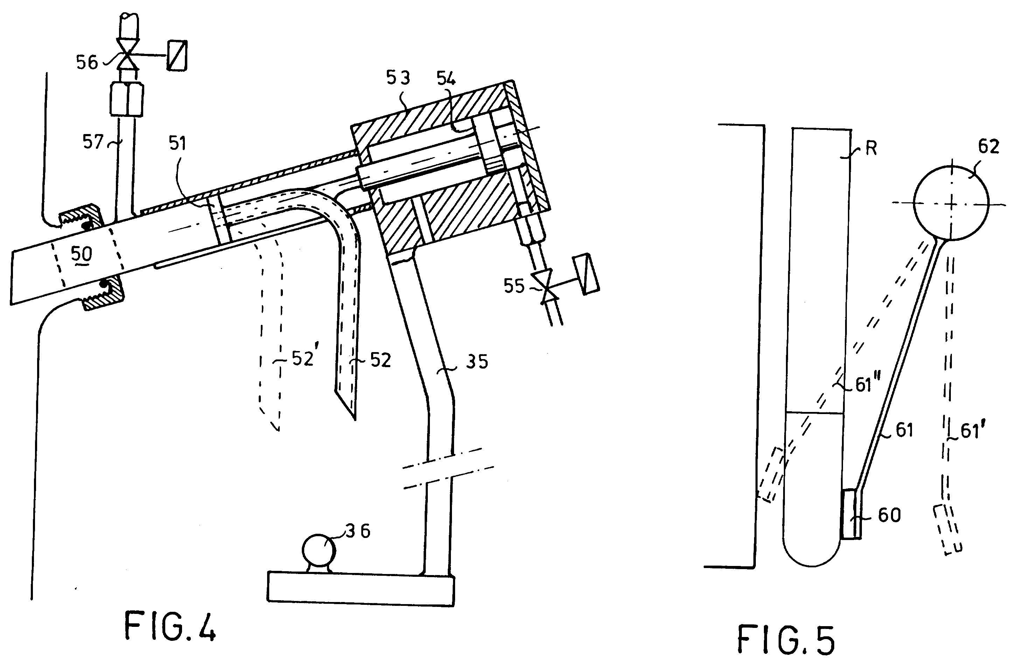

Figur 7 zeigt den Bowden-Seilzug mit der Aussenhülle 421 und dem Innenseil 422, welches mittels einer kleinen motorisch betriebenen Seiltrommel 423 angezogen werden kann, so dass der Greifer ein Reagenzglas greifen kann, nachdem er zuerst in geöffneter Lage über das Reagenzglas abgesenkt worden ist.
Die beiden Greiferhälften 420 sind innen mit einem griffigen Belag aus Gummi oder Kunstgummi versehen.
Ein wichtiger Bestandteil der Anlage bildet die ferngesteuerte Probe-Entnahmevorrichtung 5, die in Figur 4 dargestellt ist. Sie umfasst ein in die Wand des Fermentes 1 eingelassenes Rückschlagventil 50, das sich dadurch öffnen lässt, dass ein Hohlkolben 51 mit dem damit verbundenen, gekrümmten Auslassrohr 52 gegen das Ventil 50 gedrückt wird. Dabei wird automatisch eine Abdichtung zwischen dem Hohlkolben und dem Ventil hergestellt. Ein solches Ventil ist an sich bekannt. Hier ist es zusätzlich mit einem Druckluftantrieb zusammengebaut. Der Druckluftantrieb umfasst einen Zylinder 53 mit einem Kolben 54. Für die Betätigung sorgt ein ferngesteuertes Druckluftventil 55. In Ablassstellung, in der Flüssigkeit aus dem Fermenter austritt, befindet sich das Auslassrohr in der durch unterbrochene Linien angedeuteten Lage 52'.
Beim Ablassen einer Probe befindet sich ein vom Läufer herangeführtes Reagenzglas unter dem Auslassrohr. Von der Hebevorrichtung wird es während des Ablassvorganges auf- und abwärts entlang einer Kapazitäts-Sonde 60 bewegt (Figur 5). Die Kapazitäts-Sonde ist in der Lage festzustellen, ob der erwünschte Füllungsgrad (etwa 80%) erreicht ist, wonach auf Grund davon der Entnahmevorgang stoppt. Die Sonde 60 ist an einem Schwenkhebel 61 befestigt, der in Ruhelage vertikal hängt 61'. Befindet sich infolge einer Fehlmanipulation keine Reagenzglas unter dem Ablassrohr 52, schwenkt der Hebel in die Lage 61". Der Hebel ist mit einem Winkel-Potentiometer 62 verbunden, der diese Störungslage anzeigt und für den Stop der Anlage sorgt.

Nach jedem Probe-Entnahmevorgang kehrt das Auslassrohr 52 in die in Figur 4 dargestellte Ruhelage zurück, beispielsweise unter dem Einfluss einer Rückzugfeder. In dieser Lage wird nach jedem Entnahmevorgang die Entnahmevorrichtung mit Dampf gereinigt. Dazu ist die Entnahmevorrichtung mit einem ferngesteuerten Ventil 56 ausgerüstet, das durch die Leitung 57 die Entnahmevorrichtung kurzzeitig mit Dampf durchspült. Der verunreinigte Dampf tritt aus dem Rohr 52 aus und wird von einem wegschwenkbaren Auffangtrichter mit einem Schlauch 58 in einen Auffangbehälter 59 aufgefangen (Figur 1).

Fest mit der Probe-Entnahmevorrichtung 5 ist mittels eines Armes 35 eine Kugel 36 gehalten, die Teil eines Kugelgelenkes bildet, um welches die Schiene 3 schwenkbar ist. Die Kugel befindet sich senkrecht unter der Entnahmevorrichtung.

Im Folgenden werden die Vorgänge bei der Entnahme und Aufbewahrung einer Probe im Detail beschrieben. Die Anlage umfasst eine programmierbare Steuereinheit, welche die Steuersignale von den einzelnen Anlageteilen empfängt und die entsprechenden Antriebseinheiten ein- oder ausschaltet. Die Intervalle zwischen den jeweiligen Probeentnahmen sind programmierbar nach verschiedenen Prozess-Parametern.

Zu Beginn befindet sich der Läufer 4 mit angehobener Hebevorrichtung über der Entnahmeöffnung 29 des Aufbewahrungsbehälters. Der Greifer 42 befindet sich genau oberhalb eines leeren Reagenzglases. Die Hebevorrichtung 41 wird abgesenkt, der Greifer 42 schiebt sich über ein leeres Reagenzglas R, wird geschlossen und greift das leere Reagenzglas. Nun wird die Hebevorrichtung angehoben bis das Reagenzglas sich über dem Behälter 2 befindet. Der Läufer mit dem leeren Reagenzglas fährt nun zur Entnahmevorrichtung, wobei zugleich die Hebevorrichtung das Reagenzglas absenkt bis es sich genau unter dem Auslassrohr in dessen Entnahmestellung 52' befindet. Jetzt wird die Kapazitäts-Sonde 60 an das leere Reagenzglas R angelegt. Nun wird mittels des Druckluftantriebes das Ablassrohr in die Stellung 52' gebracht und die Probeentnahme beginnt. Sowie ein erstes Niveau, beispielsweise 10% erreicht ist, spricht die Kapazitäts-Sonde an, und eine zentrale Steuereinheit berechnet an Hand davon die erforderliche Entnahmezeit für einen 80% Füllungsgrad. Ist dieser Füllungsgrad erreicht, wird das Ablassrohr in seine Ruhelage zurückgebracht und das so gefüllte Reagenzglas vom Läufer weggefahren, angehoben, bis über den Aufbewahrungsbehälter gebracht und dort wieder in den Halter eingesetzt und die Hebevorrichtung angehoben. Nach Ablauf jeder Probeentnahme wird der Auffangtrichter unter die Entnahmevorrichtung geschwenkt und die mit der Biomasse in Berührung gewesenen Teile mit Dampf durchgeblasen.
Zum Schluss wird der Probehalter 24 um einen Schritt gedreht, so dass sich nun ein nächstes leeres Reagenzglas unter dem Greifer befindet. Die Zuordnung der Winkelposition des Halters erfolgt über eine am Ende der Schiene 3 angebrachte Sonde mittels eines Inkremental-Gebers der den Stellmotor 27 ansteuert.

Statt jede Probe-Entnahme-Anlage mit einem eigenen gekühlten Probe-Aufbewahrungsbehälter zu versehen, ist es unter Umständen auch möglich, weitere ähnlich aufgebaute Probe-Entnahme-Anlagen mit einem zentral dazu angeordneten gekühlten Aufbewahrungsbehälter zu kombinieren.

## Patentansprüche

1. Anlage mit einer Probeentnahmevorrichtung am Fermenter zur automatischen periodischen Probeentnahme aus einem Bioreaktor-Fermenter und einem Probeaufbewahrungsbehälter für Reagenzgläser mit den entnommenen Proben, dadurch gekennzeichnet, dass zwischen dem Fermenter (1) und dem gekühlten Probeaufbewahrungsbehälter (2) eine nach verschiedenen Parametern programmierbare, von einer Steuereinrichtung gesteuerte Transporteinrichtung (3,4) mit einer Transportschiene (3) und einem Läufer (4) angeordnet ist, die entsprechend einem eingegebenen Programm periodisch ein leeres Reagenzglas (R) aus dem Probeaufbewahrungsbehälter (2) entnimmt, dieses bis unter der am Fermenter angebrachten ferngesteuerten Probe-Entnahmevorrichtung (5) bringt, die Entnahme solange fortsetzt bis das Reagenzglas bis auf ein vorbestimmtes Mass gefüllt ist und danach das so gefüllte Reagenzglas zum gekühlten Aufbewahrungsbehälter (2) zurückbringt und wieder in denselben einsetzt, wobei auf der Transportschiene (3) ein ferngesteuerter Läufer (4) hin und her fahrbar geführt ist, der mit einer vertikal geführten auf- und abwärts bewegbaren, ferngesteuerten Hebevorrichtung (41) versehen ist, die an ihrem oberen Ende ein ferngesteuertes Greiforgan aufweist.

2. Anlage nach Anspruch 1, dadurch gekennzeichnet, dass mittels der Transporteinrichtung (3,4) das Reagenzglas (R) während der Probeentnahme auf- und abwärts entlang einer aussen am Reagenzglas (R) angelegten Kapazitäts-Sonde (60) bewegbar ist, welche nach einer gewissen Zeitdauer das Erreichen eines ersten Füllungsgrades anzeigt, an Hand dessen die Steuereinrichtung die zum Erreichen des gewünschten Füllungsgrades benötigte Zeit berechnet.

3. Anlage nach Anspruch 2, dadurch gekennzeichnet, dass die Kapazitäts-Sonde (60) an einem Schwenkarm (61) gehalten ist, mittels der die Sonde aus einer Ruhelage (61') bis am Regenzglas anliegend schwenkbar ist, so dass im Störungsfall, falls die Transporteinrichtung (3,4) kein Reagenzglas (R) unter die Probe-Entnahmevorrichtung gebracht hat, der Schwenkarm (61) weiter (61") schwenkt und ein zweites Steuersignal abgibt, welches den Probe-Entnahmevorgang sperrt.

4. Anlage nach Anspruch 2, dadurch gekennzeichnet, dass die Probeentnahme-Vorrichtung (5) mit einer automatischen Steuerung verbunden ist, welche diejenigen Teile (51,52), welche mit der aus dem Fermenter entnommenen Flüssigkeit in Berührung gewesen sind, nach jeder Probeentnahme mit Dampf ausspült.

5. Anlage nach Anspruch 1, dadurch gekennzeichnet, dass der gekühlte Probe-Aufbewahrungsbehälter (2) einen drehbaren Probehalter (24) enthält, in welchem Reagenzgläser (R) in kreisrunder Anordnung in regelmässigen Abständen voneinander gehalten sind, und dass ein Antrieb (26,27) vorgesehen ist, welcher den Probehalter (24) jeweils nach der Entnahme und nachfolgendem Einsetzen eines Reagenzglases um einen Schaltschritt dreht.

6. Anlage nach Anspruch 1, dadurch gekennzeichnet, dass die ferngesteuerte Probeentnahme-Einrichtung ein ferngesteuertes Probe-Entnahmeventil (50,51) mit einem Auslaufrohr (52) und ein ferngesteuertes Dampfventil (56) umfasst, und dass unter dem Auslaufrohr ein Abflussrohr (58) wegschwenkbar, fernbetätigbar angeordnet ist.

7. Anlage nach Anspruch 1, dadurch gekennzeichnet, dass die Hebevorrichtung (41) einen mit dem Läufer fest verbundenen Teil (412) und einen kulissenartig an diesem geführten auf- und abwärts bewegbaren Teil (411) umfasst, dass die Bewegung durch Auf- oder Abwickeln eines Seiles (413) bewerkstelligt wird, und dass eine am Seil anliegende Blattfeder die Seilspannung überwacht, indem sie bei nachlassender Seilspannung eine Lichtschranke freigibt, welche die Bewegung anhält.

## Claims

1. An installation with a sampling device on the fermenter for the automatic periodic sampling from a bioreactor-fermenter and a sample storage container for test tubes with extracted samples, characterised in that, between the fermenter (1) and the cooled sample storage container (2), is located a transporting device (3, 4) controlled by a control device programmable according to different parameters and which has a transport rail (3) and a runner (4), which in accordance with an input program periodically removes an empty test tube (R) from the sample storage container (2), brings it below the remotely controlled sampling device (5) fitted to the fermenter, maintains removal until the test tube is filled to a predetermined level and then returns the thus filled test tube to the cooled storage container (2) and reinserts the test tube therein, the remotely controlled runner (4) being guided backwards and forwards on the transport rail (3) and being provided with a vertically guided, remotely controlled lifting device (41), which can be moved up and down and which has at its upper end a remotely controlled gripping member.

2. An installation according to claim 1, characterised in that, by means of the transport device (3, 4), the test tube (R) is movable during sampling up and down along a capacity probe (60) externally applied to the test tube (R) and which after a certain time indicates the attainment of a first filling level, by means of which the control device calculates the time necessary until the predetermined filling level is reached.

3. An installation according to claim 2, characterised in that the capacity probe (60) is held on a swivel arm (61) by means of which the probe can be pivoted out of an inoperative position (61') until it engages on the test tube, so that in the case of a fault, if the transport device (3, 4) has brought no test tube (R) below the sampling device, the swivel arm (61) swivels further (61") and delivers a second control signal, which blocks the sampling process.

4. An installation according to claim 2, characterised in that the sampling device (5) is connected to an automatic control, which after each sampling operation flushes with steam those parts (51, 52) which have been in contact with the liquid removed from the fermenter.

5. An installation according to claim 1, characterised in that the sample storage container (2) contains a rotary sample holder (24), in which test tubes (R) in a circular arrangement are maintained with regular reciprocal spacings, and in that a drive (26, 27) is provided, which rotates the sample holder (24) by an indexing step following removal and subsequent insertion of a test tube.

6. An installation according to claim 1, characterised in that the remotely controlled sampling device comprises a remotely controlled sample removal valve (50, 51) with an outlet tube (52) and a remotely controlled steam valve (56), and in that below the outlet tube a discharge tube (58) is arranged in remotely controlled, pivotable away manner.

7. An installation according to claim 1, characterised in that the lifting device (41) has a part (412) secured to the runner and a part (411), which can be moved up and down and is guided in rocker-like manner, in that the movement is brought about by the winding on or off of a cable (413) and in that a leaf spring engaging the cable monitors the cable tension in order to free a light barrier, which stops the movement, when the cable tension decreases.

## Revendications

1. Installation munie d'un dispositif d'échantillonnage sur fermenteur pour l'échantillonnage automatique et périodique du contenu d'un bioréacteur-fermenteur et d'un récipient de stockage d'échantillon pour des tubes à essai avec les échantillons prélevés, caractérisée en ce que, entre le fermenteur (1) et le récipient pour le stockage d'échantillon (2) refroidi est placé un dispositif de transport (3,4) commandé par un appareil de commande et ayant une bande transporteuse (3) et une roue motrice (4), qui prélève périodiquement en fonction d'un programme préchargé un tube à essai vide (R) du récipient de stockage d'échantillon (2), apporte celui-ci jusqu'en dessous du dispositif d'échantillonnage (5) télécommandé, placé près du fermenteur, effectue le prélèvement jusqu'à ce que le tube à essai soit rempli jusqu'à un niveau prédéterminé, et ensuite ramène le tube à essai ainsi rempli dans le récipient de stockage d'échantillon refroidi (2) et le replace dans celui-ci, tandis que sur la bande transporteuse (3) est animée de façon à être mobile en va-et-vient une roue motrice télécommandée (4) qui est munie d'un dispositif de levage (41) télécommandé, guidé verticalement, mobile vers le haut et vers le bas, qui présente à son extrémité supérieure un organe de prise télécommandé.

2. Installation selon la revendication 1, caractérisée en ce qu'au moyen du dispositif de transport (3,4) le tube à essai (R) peut être déplacé vers le haut et vers le bas pendant l'échantillonnage le long d'une sonde de capacité (60) placée près du tube à essai (R) et qui après un certain laps de temps indique qu'est atteint un premier degré de remplissage, à l'appui duquel l'appareil de commande calcule le temps nécessaire pour que soit atteint le degré de remplissage souhaité.

3. Installation selon la revendication 2, caractérisée en ce que la sonde de capacité (60) est fixée à un bras orientable (61), au moyen duquel la sonde peut être orientée depuis une position de repos (61') jusqu'à la position contre le tube à essai, de telle sorte qu'en cas de perturbation, dans le cas où le dispositif de transport (3,4) n'a appporté aucun tube à essai (R) sous le dispositif d'échantillonnage, le bras orientable (61) se déplace plus loin (61") et délivre un second signal de commande, qui bloque le processus d'échantillonnage.

4. Installation selon la revendication 2, caractérisée en ce que le dispositif d'échantillonnage (5) est relié à une commande automatique, qui balaie avec de la vapeur après chaque prélèvement d'échantillon les parties (51,52) qui ont été en contact avec le fluide prélevé du fermenteur.

5. Installation selon la revendication 1, caractérisée en ce que le récipient de stockage d'échantillon refroidi (2) contient un porte-échantillon rotatif (24), dans lequel des tubes à essai (R) sont maintenus selon une disposition circulaire à des distances régulières les uns des autres, et qu'il est prévu un entraînement (26,27) qui fait tourner d'un pas de commande le porte-échantillon (24) à chaque fois après le prélèvement et l'introduction subséquente d'un tube à essai.

6. Installation selon la revendication 1, caractérisée en ce que le dispositif d'échantillonnage télécommandé comprend une vanne de prélèvement d'échantillon télécommandée (50,51) ayant un tube d'évacuation (52) et une soupape à vapeur télécommandée (56), et que sous le tube d'évacuation est disposé un tube d'écoulement (58) orientable, commandable à distance.

7. Installation selon la revendication 1, caractérisée en ce que le dispositif de levage (41) comprend une partie (412) fixée à la roue motrice et une partie (411) mobile vers le haut et vers le bas, entraînée en coulissement sur celle-ci, que le mouvement est accompli par enroulement et déroulement d'un câble (413), et qu'un ressort à lames ajusté sur le câble contrôle la tension du câble en libérant, lorsque la tension du câble d'affaiblit, une barrière photoélectrique qui arrête le mouvement.
